# EUROPÄISCHE PATENTANMELDUNG

(11) **EP 0 801 061 A2**
(43) Veröffentlichungstag der Anmeldung: **15.10.1997**
(21) Anmeldenummer: 97250111.8
(22) Anmeldetag: 19.03.1991
(51) Int. Cl.: C07D 213/46, C07D 213/40, C07D 213/60, C07C 59/48, A61K 31/44, A61K 31/19

(54) **Leukotrien-B4-Derivate, Verfahren zu ihrer Herstellung und ihre Verwendung als Arzneimittel**

(30) Priorität: 19.03.1990 DE 4009117
(62) Teilanmeldung aus: 91905964.2
(71) Anmelder: SCHERING AKTIENGESELLSCHAFT, 13342 Berlin (DE)
(72) Erfinder: Heindl, Josef, Dr., 14129 Berlin (DE); Skuballa, Werner, Dr., 13465 Berlin (DE); Buchmann, Bernd, Dr., 10557 Berlin (DE); Ekerdt, Roland, Dr., 13469 Berlin (DE); Giesen, Claudia, Dr., 13587 Berlin (DE)

(57) **Zusammenfassung**

Leukotrien-B₄-Analoge der Formel I, 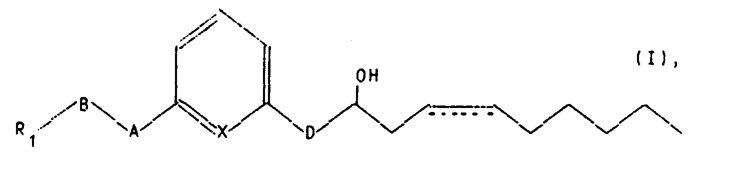 worin
einer (C₁-C₄)-Alkylgruppe oder R¹ den Rest CH₂OH,
- B: eine Alkylengruppe mit 1-3 C-Atomen in der Kette, einen Rest 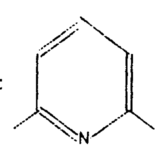 oder einen Rest 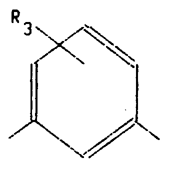 mit R³ in der Bedeutung eines Wasserstoffatoms, einer Carboxy- oder Methoxycarbonyl-Gruppe,
- A: die Gruppen 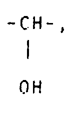 -O-, 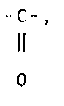 -NH-CO-, -CO-NH-, -OCH₂-, -CH=CH-, -C≡C-, -COCH₂-,
oder -CHOH-CH₂-,
- X: N oder CH,
- D: die Gruppen 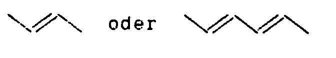 und
- .....: eine Einfach- oder Doppelbindung bedeuten, sowie gegebenenfalls deren Salze mit physiologisch unbedenklichen Basen, Verfahren zu ihrer Herstellung und ihre pharmazeutische Verwendung.

## Beschreibung

Die Erfindung betrifft neue Leukotrien-B₄-Derivate, Verfahren zu ihrer Herstellung sowie ihre Verwendung als Arzneimittel.

Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

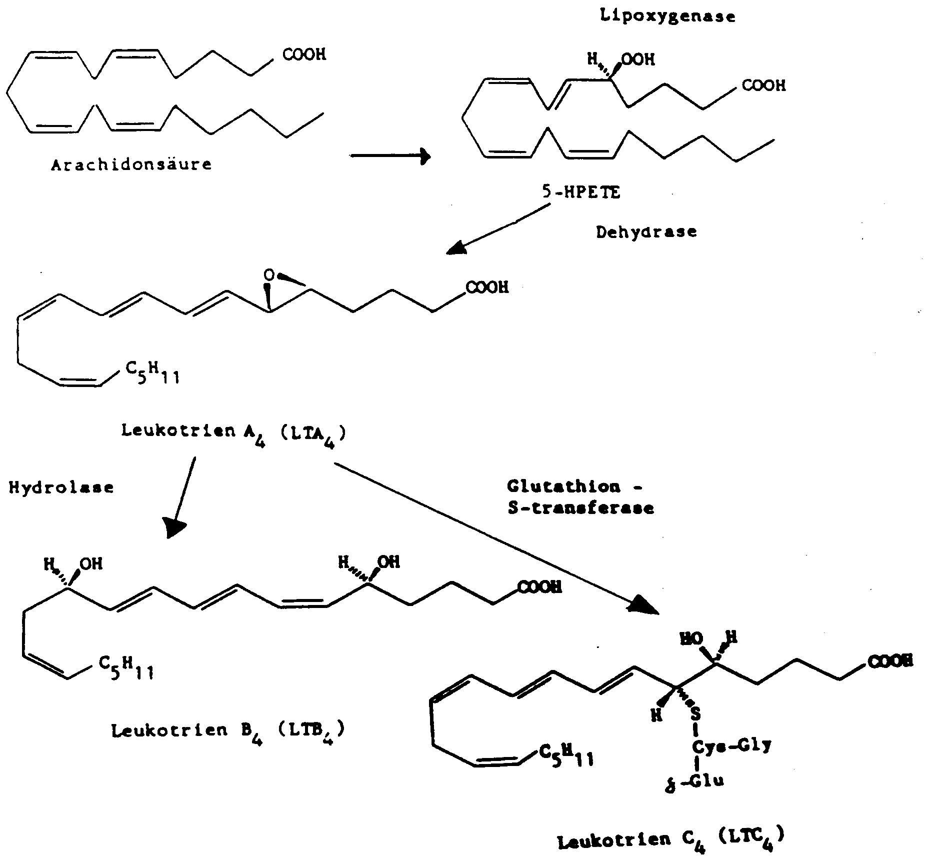

Die Nomenklatur der Leukotriene kann folgenden Arbeiten entnommen werden:
a) B. Samuelsson et al., Prostaglandins 19, 645 (1980); 17, 785 (1979).
b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzundungsmediator für entzundliche Erkrankungen ist, bei denen Leukozyten in das erkrankte Gewebe einwandern.

Vom LTB₄ weiß man, daß es die Adhäsion von Leukozyten an die Blutgefäßwand verursacht. LTB₄ ist chemotaktisch wirksam, d.h. es löst eine gerichtete Wanderung von Leukozyten in Richtung eines Gradienten steigender Konzentration aus. Außerdem verandert es aufgrund seiner chemotaktischen Aktivität indirekt die vasculäre Permeabilität, wobei ein Synergismus mit Prostaglandin E₂ beobachtet wurde. LTB₄ spielt offensichtlich bei entzundlichen, allergischen und immunologischen Prozessen eine entscheidende Rolle.

Leukotriene und insbesondere LTB₄ sind an Hauterkrankungen beteiligt, die mit entzundlichen Prozessen (erhöhte Gefaßpermeabilität und Ödembildung, Zellinfiltration), erhöter Proliferation der Hautzellen und Juckreiz einhergehen, wie beispielsweise bei Ekzemen, Erythemen, Psoriasis, Pruritus und Akne. Pathologisch erhöhte Leukotrienkonzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.

Weiterhin sind Leukotriene und LTB₄ insbesondere bei Arthritis, chronischer Lungenerkrankung (z.B. Asthma), Rhinitis und entzundlichen Darmerkrankungen beteiligt.

Antagonisten gegen LTB₄ selbst oder Inhibitoren jener Enzyme, die an der Synthese des LTB₄ beteiligt sind, können als spezifische Heilmittel, besonders gegen Krankheiten, die mit Entzündungen und allergischen Reaktionen einhergehen, wirksam sein.

Neben therapeutischen Moglichkeiten, die sich aus einer Antagonisierung des LTB₄ mit LTB₄-Analoga ableiten lassen, konnte kürzlich auch die Nutzlichkeit und potentielle Anwendung von Leukotrien B₄-Agonisten zur Behandlung von Pilzerkrankungen der Haut gezeigt werden (H. Katayama, Prostaglandins 34, 797 (1988)).
Weitere Anwendungen für LTB₄-Agonisten ergeben sich aus der LTB₄-stimulierten Aktivierung von Komponenten des Immunsystems bei verschiedenen Indikationen, wie Infektionserkrankungen, Brandverletzungen, in der Tumortherapie oder z.B. in der AIDS-Therapie. Bei AIDS-Kranken wurde z.B. eine verminderte Freisetzung von LTB₄ bei der Stimulation von Neutrophilen berichtet. (AIDS, 1989, 3, 651).

Die Erfindung betrifft neue Leukotrien-B₄-Analoge der Formel I, 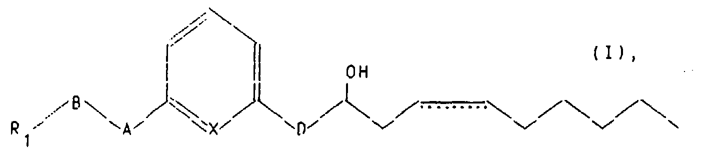 worin
- R¹: den Rest COOR² mit R² in der Bedeutung eines Wasserstoffatoms oder einer
(C₁-C₄)-Alkylgruppe oder R¹ den Rest CH₂OH.
- B: eine Alkylengruppe mit 1-3 C-Atomen in der Kette, einen Rest 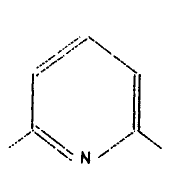 oder einen Rest 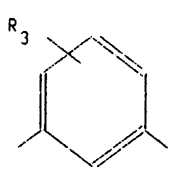 mit R₃ in der Bedeutung eines Wasserstoffatoms, einer Carboxy- oder Alkoxycarbonyl-Gruppe mit 1-4 C-Atomen im Alkoxyrest,
- A: die Gruppen 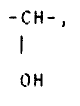 -O-, 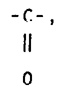 -NH-CO-, -CO-NH-, -OCH₂-, -CH=CH-, -C≡C-, -COCH₂-,
oder -CHOH-CH₂-,
- X: N oder CH,
- D: die Gruppen 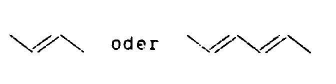 und
- .....: eine Einfach- oder Doppelbindung bedeuten,
sowie gegebenenfalls deren Salze mit physiologisch unbedenklichen Basen.

Als Alkylgruppen R₂ kommen gerad- oder verzweigtkettige Alkylgruppen mit 1-4 C-Atomen in Betracht, wie beispielsweise Methyl, Ethyl, Propyl, Isopropyl, Butyl, Isobutyl, tert.-Butyl. Als bevorzugte Alkylgruppen R₂ sind solche mit 1-2 C-Atomen zu nennen.

Als Alkylengruppe B kommen geradkettige oder verzweigtkettige, gesättigte Alkylenreste mit 1-3 C-Atomen, in Frage. Beispielsweise seien genannt: Die Methylen, die Ethylen, die 1,2-Propylen und insbesondere die Trimethylengruppe.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Leukotrien-B₄-Derivate der Formel I bilden mit α-, β-, γ,-Cyclodextrin Cyclodextrinclathrate.

Besonders bevorzugte Verbindungen der vorliegenden Erfindung sind Verbindungen der Formel I, worin
- R¹: COOH, COOCH₃ oder COOC₂H₅,
- B: -(CH₂)ₙ mit n=1-3,
- A: die Gruppen 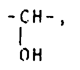 -O-, 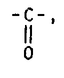 -NH-CO-, -CO-NH-, -OCH₂-, -CH=CH-, -C≡C-, -COCH₂-,
oder -CHOH-CH₂-,
- X: N oder CH,
- D: die Gruppen 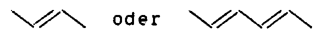 und
- .....: eine Einfachbindung darstellt.

Außerdem betrifft die Erfindung ein Verfahren zur Herstellung der erfindungsgemäßen Leukotrien-B₄-Derivate der Formel I, das dadurch gekennzeichnet ist, daß man in an sich bekannter Weise
a) eine Verbindung der Formel II 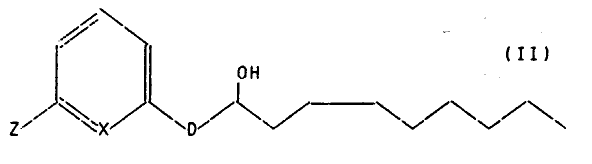 worin D die oben angegebene Bedeutung hat und .... eine Einfach-oder eine Doppelbindung und X Stickstoff darstellen und worin Z ein Bromatom oder ein Iodatom symbolisiert, nach Umsetzung mit n-Butyllithium mit einer Verbindung der Formel III

   R¹-B-R⁴ (III),

   worin R¹ und B die oben angegebenen Bedeutungen haben und R⁴ die Reste -CHO oder -COOCH₃ bedeutet, umsetzt, oder
b) eine Verbindung der Formel IV, 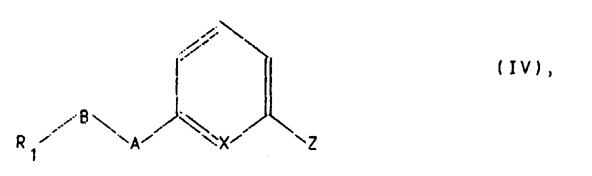 worin R¹, B, A und Z die oben angegebenen Bedeutungen haben und X Stickstoff bedeutet, mit einem Tri-n-butylstannan der Formel V, 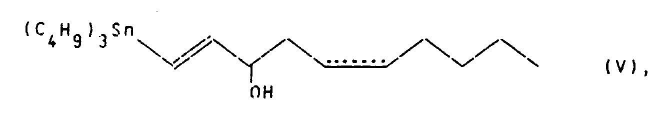 worin ..... eine Einfach- oder eine Doppelbindung darstellt, in Gegenwart eines Palladium-Katalysators, wie zum Beispiel 1,1'-Bis-(diphenylphosphino)-ferrocen-palladium-II-chlorid umsetzt,
   oder
c) ein Phenylenderivat der Formel VI, 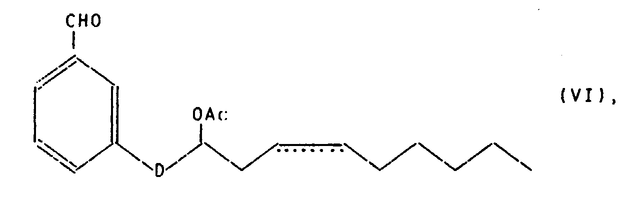 worin D die Gruppen 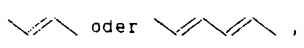 Ac eine Acylgruppe mit bis zu 8 C-Atomen und ... eine Einfach- oder eine Doppelbindung bedeuten, mit einer Grignardverbindung der allgemeinen Formel VII 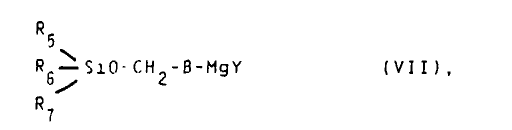 worin B die obengenannte Bedeutung besitzt, Y ein Chloratom oder ein Bromatom darstellt und die Substituenten R₅, R₆ und R₇ gleich oder verschieden sind und C₁-C₄-Alkylgruppen oder Phenylgruppen bedeuten, umsetzt, die freie Hydroxygruppe acyliert, den Silylether abspaltet und gewunschtenfalls die Hydroxymethylverbindung zur Carboxylverbindung oxidiert, sowie vorhandene Acylgruppen abspaltet und die Carboxylgruppe verestert oder in ihre Salze überführt.

Die Ausgangsverbindungen II, IV und VI werden entsprechend den in den Beispielen angegebenen Verfahren hergestellt.

Die Umsetzung der Verbindungen der Formel II mit Verbindungen der Formel III erfolgt in an sich bekannter Weise wie in den betreffenden Beispielen angegeben. Die darin angegebenen Reaktionsbedingungen sind, was Losungsmittel, Temperatur und Reaktionszeit betrifft, natürlich nicht nur auf die Angaben in Beispielen begrenzt, sondern können in einem für den Durchschnittsfachman nachvollziehbaren Rahmen abgewandelt werden. Das gleiche gilt für die Umsetzung von IV mit V oder VI mit VII.

Als Silyletherschutzgruppen des Grignardreagenz der Formel VII wird vorzugsweise die tert.-Butyl-dimethylsilylgruppe oder die tert.-Butyldiphenylsilylgruppe verwendet.

Die Oxidation der Hydroxygruppen wird nach den dem Fachmann bekannter Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation.

Die Oxidation mit Pyridiniumchromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40°C bis +40°C, vorzugsweise -30°C bis 0°C in Aceton als Lösungsmittel ausgeführt.

Die Oxidation mit Platin/Sauerstoff wird bei Temperaturen von 0°C bis 60°C, vorzugsweise 20°C bis 40°C in einem gegen das Oxidationsmittel inerten Losungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen in einer wässrigen Lösung einer organischen Saure, wie z.B. Oxalsäure, Essigsäure, Propionsäure u.a., oder in einer wässrigen Lösung einer anorganischen Säure, wie z. B. Salzsäure, durchgeführt. Zur Verbesserung der Loslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder in der wässrigen Losung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10°C bis +70°C, vorzugsweise bei +25°C.

Die Leukotrien-B₄-Derivate der Formel I mit R₂ in der Bedeutung eines Wasserstoffatoms können mit geeigneten Mengen der entsprechenden anorganischen Basen unter Neutralisierung in ein Salz überführt werden. Beispielsweise erhalt man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Enthält das Ausgangsprodukt OH-Gruppen im Leukotrien-B₄-Rest so werden diese OH-Gruppen auch zur Reaktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Der Ersatz der chemisch und metabolisch labilen cis-Δ^{6,7}-Doppelbindung und der trans-Δ^{8,9}-Doppelbindung des LTB₄ durch einen 1,3-substituierten Phenyl- bzw. 2,6-substituierten Pyridylring führt zu stabileren leukotrienen mit einem Phenyl- bzw. Pyridylring in 6,9-Stellung. Die Verbindungen der allgemeinen Formel I sind je nach Bedeutung der einzelnen Reste R₁, B, A, D und der Derivatisierung der funktionellen Gruppen entweder
a) chemisch stabile LTB₄ Agonisten oder
b) chemisch stabile LTB₄ Antagonisten.

Mit den Verbindungen der Formel I, die Rezeptoragonisten darstellen, kann die LTB₄-Rezeptor vermittelte stimulierte Aktivierung von Komponenten des Immunsystems bei verschiedenen Indikationen, wie Infektionserkrankungen, Mykosen, Brandverletzungen, in der Tumortherapie oder z.B. in der AIDS-Therapie therapeutisch genutzt werden. Bei AIDS-Kranken wurde z.B. eine verminderte Freisetzung von LTB₄ bei der Stimulation von Neutrophilen berichtet.

Die Verbindungen der Formel I, die Rezeptorantagonisten darstellen, wirken antientzundlich und antiallergisch. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Diese neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen.

Die neuen Leukotrien-B₄-Derivate eignen sich auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Die neuen Leukotrien-B₄-Derivate, die LTB₄-Antagonisten darstellen, können auch in Kombination, wie z.B mit Lipoxygenasehemmern, Cyclooxygenasehemmern, Prostacyclinagonisten, Thromboxanantagonisten, Leukotrien D₄-antagonisten, Leukotrien E₄-antagonisten, Leukotrien F₄-antagonisten, Phosphodiesterasehemmern oder PAF-Antagonisten verwendet werden.

Die Herstellung der Arzneimittelspezialitäten erfolgt in üblicher Weise, indem man die Wirkstoffe mit geeigeneten Zusätzen in die gewünschte Applikationsform, wie zum Beispiel: Losungen, Lotionen, Salben, Cremes oder Pflaster, überfuhrt. In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Die nachfolgenden Ausführungsbeispiele dienen zur Erläuterung des erfindungsgemäßen Verfahrens.

### BEISPIEL 1

### (5RS)-5-Hydroxy-5-{6-[(1E)-(3RS)-3-hydroxy-1-undecenyl]-2-pyridyl}-pentansäure-methylester

A. Eine Losung von 516 mg 2,6-Dibrompyridin in 4 ml Dimethylformamid wird mit 1 g (1E)-1-(Tri-n-butylstannyl)-1-undecen-(3RS)-3-ol und 81 mg 1,1'-Bis-(diphenylphosphino)-ferrocen-palladium-II-chlorid versetzt und unter Argonatmosphäre 24 Stunden bei Raumtemperatur gerührt. Die Reaktionsmischung wird in 20 ml 5%ige Salzsäure gegossen, mit Diethylether ausgeschüttelt, die organische Phase mit Natriumsulfat getrocknet und eingeengt. Der Rückstand wird an Kieselgel mit Hexan/Ethylacetat/Triethylamin 95/5/1 chromatographiert. Man erhält so 355 mg 2-Brom-6-[(1E)-(3RS)-3-hydroxy-1-undecenyl]-pyridin als farbloses Öl.
   - IR (CHCl₃):: 2925, 2860, 1655, 1575, 1548, 1432, 1120, 985 cm⁻¹.

   Die in Beispiel 1A verwendete Organozinnverbindung ist in der deutschen Patentanmeldung P 3909326.3 beschrieben.
B. Eine Losung von 326 mg 2-Brom-6-[(1E)-(3RS)-3-hydroxy-1-undecenyl]-pyridin in 5 ml Tetrahydrofuran wird bei -78°C unter Rühren und Argonatmosphäre mit 1,25 ml n-Butyllithium (1,6 molar in Hexan) versetzt. Nach 10 Minuten wird eine Lösung von 5-Oxo-pentansäure-methylester in 1 ml Tetrahydrofuran bei -78°C hinzugefügt und 2 Stunden bei dieser Temperatur gerührt. Die Reaktionsmischung wird mit Wasser versetzt, mit Dichlormethan ausgeschüttelt, über Natriumsulfat getrocknet, eingeengt und das Rohprodukt an Kieselgel mit Hexan/Ethylacetat 98/2 chromatographiert. Man erhält so 188 mg der Titelverbindung als farbloses Öl.
   - IR (CHCl₃):: 3600, 2925, 2860, 1730,1574, 1455, 1260, 1010cm⁻¹.

### BEISPIEL 2

### (5RS)-5-Hydroxy-5-{6-[(1E)-(3RS)-3-hydroxy-1-undecenyl]-2-pyridyl}-pentansäure

Eine Lösung von 140 mg (5RS)-5-Hydroxy-5-{6-{(1E)-(3RS)-3-hydroxy-1-undecenyl]-2-pyridyl}-pentansäuremethylester in 6 ml Methanol wird mit 6 ml 0,5 n Natronlauge versetzt und 2 Stunden bei Raumtemperatur gerührt. Das Methanol wird im Vakuum entfernt, der Rückstand mit 0,5 n Schwefelsäure auf pH 4 angesäuert, mit Ethylacetat ausgeschüttelt, die organische Phase über Natriumsulfat getrocknet und eingeengt. Man erhält so 125 mg der Titelverbindung als farbloses Öl.
- IR:: 3370, 2960, 2922, 2852, 1710,1570, 1455, 1260, 1080, 1020, 800 cm⁻¹.

### BEISPIEL 3

### 2-[(1RS)-1-Hydroxy-1-(3-methoxycarbonylphenyl)-methyl]-6-[(1E)-(3RS)-3-hydroxy-1-undecenyl]-pyridin

Unter den Bedingungen des Beispiels 1B werden 326 mg 2-Brom-6-[(1E)-(3RS)-3-hydroxy-1-undecenyl]-pyridin mit 1,38 ml n-Butyllithium (1,6 molar in Hexan) und 181 mg 3-Formylbenzoesäuremethylester (J. Org. Chem. 31, 1966 2585) umgesetzt, aufbereitet und an Kieselgel mit Hexan/Ethylacetat 8/2 chromatographiert. Es werden 105 mg der Titelverbindung als farbloses Öl erhalten.
- IR:: 2030, 2860, 1720, 1290, 1095 cm⁻¹.

### BEISPIEL 4

### 2-[(1RS)-1-Hydroxy-1-(3-carboxyphenyl)-methyl]-6-[(1E)-(3RS)-3-hydroxy-1-undecenyl]-pyridin

Unter den Bedingungen des Beispiels 2 werden 80 mg 2-[(1RS)-1-Hydroxy-1-(3-methoxycarbonylphenyl)-methyl]-6-[(1E)-(3RS)-3-hydroxy-1-undecenyl]-pyridin in 4 ml Methanol mit 4 ml 0,5 n Natronlauge verseift und aufbereitet. Es werden 40 mg der Titelverbindung als farbloser Schaum erhalten.
- IR:: 3400, 2925, 2855, 1695, 1570, 1455, 1262, 970, 750 cm⁻¹.

### BEISPIEL 5

### 3-{6-[(1E)-(3RS)-3-Hydroxy-1-undecenyl]-2-pyridyloxy}-benzoesäuremethylester

A. Eine Lösung von 2,37 g 2,6-Dibrompyridin und 1,52 g 3-Hydroxybenzoesäuremethylester in 10 ml Dimethylformamid wird mit 6,6 g Cäsiumcarbonat versetzt und unter Rühren 3 Stunden auf 120°C erhitzt. Die Reaktionsmischung wird über Kieselgur filtriert, mit Dichlormethan nachgewaschen und das Filtrat im Vakuum eingeengt. Der Rückstand wird bei 140-150°C und 0,04 mbar am Kugelrohr destilliert. Es werden 2,98 g 3-(6-Brom-2-pyridyloxy)-benzoesäuremethylester vom Schmelzpunkt 68-69°C erhalten.
   - IR (CHCl₃):: 2925, 1720, 1580, 1560, 1420, 1285, 1100 cm⁻¹.
B. Eine Lösung von 617 mg 3-(6-Brom-2-pyridyloxy)-benzoesäuremethylester in 4 ml Dimethylformamid wird mit 1,09 g (1E)-1-(Tri-n-butylstannyl)-1-undecen-(3RS)-3-ol und 74 mg 1,1'-Bis-(diphenylphosphino)-ferrocen-palladium-II-chlorid versetzt und unter Argonatmosphäre 48 Stunden bei Raumtemperatur gerührt. Die Reaktionsmischung wird an Kieselgel mit Hexan/Ethylacetat = 9/1 chromatographiert. Es werden 340 mg Rohprodukt als gelbes Öl erhalten, das zur vollständigen Reinigung der Hochdruck-Flüssigkeits-Chromatographie an silanisiertem Kieselgel (RP 18-Material) mit Methanol/Wasser = 9/1 unterworfen wird. Man erhält so 160 mg der Titelverbindung als farbloses Öl.
   - IR (CHCl₃):: 3605, 2930, 1722, 1590, 1570, 1435, 1260, 1100, 1015 cm⁻¹.

### BEISPIEL 6

### 3-{6-[(1E)-(3RS)-3-Hydroxy-1-undecenyl]-2-pyridyloxy}-benzoesäure

Unter den Bedingungen des Beispiels 2 werden 40 mg 3-{6-[(1E)-(3RS)-3-Hydroxy-1-undecenyl]-2-pyridyloxy}-benzoesäuremethylester in 1 ml Methanol mit 1 ml 1 n Natronlauge verseift und aufbereitet. Es werden 29 mg der Titelverbindung vom Schmelzpunkt 85-87°C erhalten.
- IR (CHCl₃):: 2930,1710, 1590, 1570, 1434, 1260, 1095, 1012 cm⁻¹.

### BEISPIEL 7

### 4-{6-[(1E)-(3RS)-3-Hydroxy-1-undecenyl]-2-pyridylcarbonylamino}-buttersäure-ethylester

A. 4 g 6-Brompyridin-2-carbonsäure und 25 g Thionylchlorid werden 1 Stunde unter Rückfluß erhitzt. Das überschüssige Thionylchlorid wird im Vakuum entfernt und der Rückstand am Kugelrohr bei 110°C und 0,04 mbar destilliert. Das so erhaltene 6-Brompyridin-2-carbonsäurechlorid wird Zusammen mit 3,32 g 4-Aminobuttersäureethylester, Hydrochlorid in 40 ml Dioxan gelost, unter Eiskühlung mit 6 g Triethylamin versetzt und 5 Stunden bei Raumtemperatur gerührt. Die Reaktionsmischung wird auf Wasser gegeben, mit Diethylether extrahiert, getrocknet (Natriumsulfat) und eingeengt. Der Rückstand wird am Kugelrohr bei 170°C und 0,04 mbar destilliert und man erhalt so 3,2 g 4-(6-Brom-2-pyridylcarbonylamino)-buttersäureethylester als hellgelbes Öl.
   - IR (CHCl₃):: 3400, 2930, 1720, 1675, 1520, 1425, 1300 cm⁻¹.
B. Unter den Bedingungen des Beispiels 5B werden 630 mg 4-(6-Brom-2-pyridylcarbonylamino)-buttersäureethylester und 1,007 g (1E)-1-(Tri-n-butylstannyl)-1-undecen-(3RS)-3-ol in 4 ml Dimethylformamid in Gegenwart von 71 mg 1,1'-Bis-(diphenylphosphino)-ferrocen-palladium-II-chlorid als Katalysator umgesetzt und die Reaktionsmischung an Kieselgel mit Hexan/Ethylacetat = 95/5 bis 8/2 chromatographiert. Es werden 300 mg der Titelverbindung als farbloses Öl erhalten.
   - IR (CHCl₃):: 3390, 2925, 2860, 1725, 1670, 1525, 1450, 1260 cm⁻¹.

### BEISPIEL 8

### 4-{6-[(1E)-(3RS)-3-Hydroxy-1-undecenyl]-2-pyridylcarbonylamino}-buttersäure

Unter den Bedingungen des Beispiels 2 werden 60 mg 4-{6-[(1E)-(3RS)-3-Hydroxy-1-undecenyl]-2-pyridylcarbonylamino}-buttersäureethylester in 1,5 ml Methanol mit 1,5 ml 1 n Natronlauge verseift und aufbereitet. Es werden 35 mg der Titelverbindung als farbloses Öl erhalten.
- IR (CHCl₃):: 3390, 2925, 2860, 1722, 1670, 1525, 1450, 1260 cm⁻¹.

### BEISPIEL 9

### 5-{6-[(1E)-(3RS)-3-Hydroxy-1-undecenyl]-2-pyridylamino}-5-oxo-pentansäure-methylester

A. Eine Losung von 1,0 g 2-Amino-6-brompyridin und 580 mg Triethylamin in 12 ml Tetrahydrofuran wird unter Rühren und Eiskühlung tropfenweise mit 0,78 ml Glutarsäuremethylesterchlorid versetzt und die Mischung 17 Stunden bei Raumtemperatur gerührt. Die Reaktionsmischung wird auf Wasser gegeben, mit Diethylether ausgeschüttelt, die organische Phase getrocknet (Natriumsulfat) und eingeengt. Der Rückstand wird an Kieselgel mit Dichlormethan/Methanol = 95/5 chromatographiert. Es werden 668 mg 5-(6-Brom-2-pyridylamino)-5-oxo-pentansäuremethylester vom Schmelzpunkt 128-131°C erhalten.
B. Unter den Bedingungen des Beispiels 5B werden 603 mg 5-(6-Brom-2-pyridylamino)-5-oxo-pentansäuremethylester und 1,01 g (1E)-1-(Tri-n-butylstannyl)-1-undecen-(3RS)-3-ol in 12 ml Dimethylformamid in Gegenwart von 71 mg 1,1'-Bis-(diphenylphosphino)-ferrocen-palladium-II-chlorid als Katalysator umgesetzt und die Reaktionsmischung an Kieselgel mit Hexan/Ethylacetat = 95/5 bis 75/25 chromatographiert. Es werden 155 mg der Titelverbindung als farbloses Öl erhalten.
   - IR (CHCl₃):: 3420, 2925, 2860, 1730, 1695, 1575, 1450, 1260, 1095, 1010 cm⁻¹.

### BEISPIEL 10

### 4-{6-[(1E)-(3RS)-3-Hydroxy-1-undecenyl]-2-pyridylamino}-5-oxo-pentansäure

Unter den Bedingungen des Beispiels 2 werden 60 mg 4-{6-[(1E)-(3RS)-3-Hydroxy-1-undecenyl]-2-pyridylamino}-5-oxo-pentansäuremethylester in 1,5 ml Methanol mit 1,5 ml 1 n Natronlauge verseift und aufbereitet. Es werden 40 mg der Titelverbindung als farbloses Öl erhalten.
- IR (CHCl₃):: 2935, 2865, 1700, 1580, 1458 cm⁻¹.

### BEISPIEL 11

### 4-{6-[(1E)-(3RS)-3-Hydroxy-1-undecenyl]-2-pyridyloxy}-essigsäuremethylester

A. Eine Suspension von 178 mg Natriumhydrid (80%ige Dispersion in Mineralöl) in 4 ml Dimethylformamid wird unter Argonatmosphäre, Rühren und Eiskühlung mit einer Lösung von 361 mg Glykolsäuremethylester in 2 ml Dimethylformamid versetzt und 3 Stunden bei Raumtemperatur gerührt. Danach wird unter Eiskühlung eine Lösung von 948 mg 2,6-Dibrompyridin in 2 ml Dimethylformamid hinzugefügt und die Mischung 48 Stunden bei Raumtemperatur gerührt. Die Reaktionsmischung wird auf Eis gegossen und mit Ethylacetat extrahiert. Die organische Phase wird 4 mal mit gesättigter Kochsalzlösung gewaschen, über Natriumsulfat getrocknet und eingeengt. Es werden 790 mg 2-(6-Brom-2-pyridyloxy)-essigsäuremethylester als öliges Rohprodukt erhalten.
B. Unter den Bedingungen des Beispiels 5B werden 780 mg des obigen Rohprodukts und 1,95 g (1E)-1-(Tri-n-butylstannyl)-1-undecen-(3RS)-3-ol in 23 ml Dimethylformamid in Gegenwart von 138 mg 1,1'-Bis-(diphenylphosphino)-ferrocen-palladium-II-chlorid als Katalysator umgesetzt. Die Reaktionsmischung wird auf Eis gegossen, mit Ethylacetat ausgeschüttelt, die organische Phase 4 mal mit gesättigter Kochsalzlösung gewaschen, über Natriumsulfat getrocknet und eingeengt. Der Rückstand wird an Kieselgel mit Hexan/Ethylacetat = 95/5 chromatographiert. Es werden 161 mg der Titelverbindung als farblose Öl erhalten.
   - IR (CHCl₃):: 2960, 2930, 2580, 1753, 1590, 1575, 1448, 1260 cm⁻¹.

### BEISPIEL 12

### 2-{6-[(1E)-(3RS)-3-Hydroxy-1-undecenyl]-2-pyridyloxy}-essigsäure

Unter den Bedingungen des Beispiels 2 werden 25 mg 2-{6-[(1E)-(3RS)-3-Hydroxy-1-undecenyl]-2-pyridyloxy}-essigsäuremethylester in 1,5 ml Methanol mit 1,5 ml 1 n Natronlauge verseift und aufbereitet. Es werden 20 mg der Titelverbindung als hellgelbes Öl erhalten.
- IR (CHCl₃):: 2960, 2930, 2857, 1735, 1588, 1574, 1448, 1260 cm⁻¹.

### BEISPIEL 13

### 2-(3-Methoxycarbonylbenzoyl)-6-[(1E)-(3RS)-3-hydroxy-1-undecenyl]-pyridin

Unter den Bedingungen des Beispiels 1B werden 652 mg 2-Brom-6-[(1E)-(3RS)-3-hydroxy-1-undecenyl]-pyridin in 3 ml Tetrahydrofuran mit 2,75 ml n-Butyllithium (1,6 molar in Hexan) und 427 mg Isophthalsäuredimethylester in 3 ml Tetrahydrofuran versetzt, aufbereitet und an Kieselgel mit Hexan/Ethylacetat = 95/5 bis 75/25 chromatographiert. Es werden 130 mg öliges Rohprodukt erhalten, das zur vollständigen Reinigung der Hochdruck-Flüssigkeits-Chromatographie an silanisiertem Kieselgel (RP 18-Material) mit Methanol/H₂O = 85/15 unterworfen wird. Man erhält so 58 mg der Titelverbindung als farbloses Ol.
- IR:: 2928, 2858, 1728, 1668, 1580, 1440, 1272, 1235, 1162, 740, 725 cm⁻¹.

### BEISPIEL 14

### 2-(3-Carboxybenzoyl)-6-[(1E)-(3RS)-3-hydroxy-1-undecenyl]-pyridin

Unter den Bedingungen des Beispiels 2 werden 20 mg 2-(3-Methoxycarbonylbenzoyl)-6-[(1E)-(3RS)-3-hydroxy-1-undecenyl]-pyridin in 1 ml Methanol mit 2 ml 1 n Natronlauge verseift und aufbereitet. Es werden 15 mg der Titelverbindung als farbloses Öl erhalten.
- IR (CHCl₃):: 3018, 2963, 2930, 1720, 1670, 1607, 1265, 1100, 1015 cm⁻¹.

### BEISPIEL 15

### 2-[3,5-Bis-(methoxycarbonyl)-benzoyl]-6-[(1E)-(3RS)-3-hydroxy-1-undecenyl]-pyridin

Unter den Bedingungen des Beispiels 1B wird 1 g 2-Brom-6-[(1E)-(3RS)-3-hydroxy 1-undecenyl]-pyridin in 10 ml Tetrahydrofuran mit 4,26 ml n-Butyllithium (1,6 molar in Hexan) und 807 mg 1,3,5-Benzoltricarbonsäuretrimethylester in 6 ml Tetrahydrofuran versetzt, aufbereitet und an Kieselgel mit Hexan/Ethylacetat = 95/5 bis 75/25 chromatographiert. Es werden 203 mg öliges Rohprodukt erhalten, das zur vollständigen Reinigung der Hochdruck-Flüssigkeits-Chromatographie an silanisiertem Kieselgel (RP 18-Material) mit Methanol/H₂O = 9/1 unterworfen wird. Man erhält so 74 mg der Titelverbindung als farbloses Öl.
- IR(Film):: 3560-3160, 2920, 2850, 1730, 1670, 1590, 1450, 1345, 875, 850, 820 cm⁻¹.

### BEISPIEL 16

### 2-[3,5-Bis-(carboxy)-benzoyl]-6-[(1E)-(3RS)-3-hydroxy-1-undecenyl]-pyridin

Unter den Bedingungen des Beispiels 2 werden 20 mg 2-[3,5-Bis-(methoxycarbonyl)-benzoyl]-6-[(1E)-(3RS)-3-hydroxy-1-undecenyl]-pyridin in 2 ml Methanol mit 2 ml 1 n Natronlauge verseift und aufbereitet. Es werden 6 mg der Titelverbindung als farbloses Öl erhalten.
- IR (CHCl₃):: 3580-3260, 3005, 1725, 1608, 1052, 1030, 1012, 930 cm⁻¹.

### BEISPIEL 17

### (5RS)-5-Hydroxy-5-{6-[(1E,3E)-(5RS)-5-hydroxy-1,3-tridecadienyl]-2-pyridyl}-pentansäuremethylester

A. Eine Lösung von 5,88 g 4-Phosphonocrotonsäuretriethylester in 60 ml Tetrahydrofuran wird unter Rühren und Argonatmosphäre bei -20°C portionsweise mit 2,5 g Kalium-tert.-butanolat versetzt. Nach 30 Minuten wird bei -20°C eine Lösung von 2,6 g 6-Brom-pyridin-2-aldehyd zugetropft und die Mischung noch 1 Stunde bei dieser Temperatur gerührt. Die Reaktionsmischung wird auf Eis gegossen, mit Diethylether ausgeschüttelt, die organische Phase mit gesättigter Kochsalzlösung gewaschen, über Natriumsulfat getrocknet und eingeengt. Der Rückstand wird an Kieselgel mit Hexan/Ethylacetat = 95/5 chromatographiert. Es werden 2,66 g 5-(6-Brom-2-pyridyl)-(E,E)-2,4-penta-diensäureethylester als Rohprodukt erhalten.
B. Eine Lösung von 2,6 g 5-(6-Brom-2-pyridyl)-(E,E)-2,4-penta-diensäureethylester in 73 ml Toluol wird unter Rühren und Argonatmosphäre bei -70°C tropfenweise mit 15,4 ml Diisobutylaluminiumhydrid (20%ige Lösung in Toluol) versetzt und die Mischung noch 45 Minuten bei dieser Temperatur gerührt. Die Reaktionsmischung wird bei -70°C nacheinander tropfenweise mit 5,5 ml Isopropanol und 7,3 ml Wasser versetzt, 1 Stunde bei Raumtemperatur gerührt, über Kieselgur abgesaugt, mit Dichlormethan nachgewaschen, das Filtrat über Natriumsulfat getrocknet und eingeengt. Es werden 2,2 g 5-(6-Brom-2-pyridyl)-(E,E) -2,4-penta-dien-1-ol als Rohprodukt erhalten.
C. Eine Lösung von 2 g 5-(6-Brom-2-pyridyl)-penta-(E,E)-2,4-dien-1-ol in 32 ml Dichlormethan wird mit 5,8 g Mangandioxid 2 Stunden kräftig gerührt. Die Reaktionsmischung wird über Kieselgur abgesaugt, eingeengt und der Rückstand an Kieselgel mit Dichlormethan chromatographiert. Es werden 1,17 g 5-(6-Brom-2-pyridyl)-(E,E)-2,4-penta-dienaldehyd als Rohprodukt erhalten.
D. Zu einer Lösung von 1,17 g 5-(6-Brom-2-pyridyl)-(E,E)-2,4-penta-dienaldehyd in 25 ml Diethylether wird unter Rühren und Argonatmosphäre bei -20°C eine Lösung von Octylmagnesiumbromid (hergestellt aus 150 mg Magnesium in 5 ml Diethylether und 1,14 g Octylbromid in 2,5 ml Diethylether) getropft. Nach 1,5 Stunden bei -20°C wird die Reaktionsmischung mit 15 ml gesättigter Ammoniumchloridlösung versetzt, mit Diethylether ausgeschüttelt über Natriumsulfat getrocknet, eingeengt und der Rückstand an Kieselgel mit Hexan/Ethylacetat = 95/5 chromatographiert. Es werden 477 mg 2-Brom-6-[(1E,3E)-(5RS)-5-hydroxy-1,3-tridecadienyl]-pyridin als Rohprodukt erhalten.
E. Unter den Bedingungen des Beispiels 1B werden 470 mg 2-Brom-6-[(1E,3E)-(5RS)-5-hydroxy-1,3-tridecadienyl]-pyridin in 7 ml Tetrahydrofuran mit 1,84 ml n-Butyllithium (1,6 molar in Hexan) und 200 mg 5-Oxo-pentansäuremethylester in 1,5 ml Tetrahydrofuran versetzt, aufbereitet und an Kieselgel mit Hexan/Ethylacetat = 95/5 chromatographiert. Es werden 29 mg der Titelverbindung als farbloses Öl erhalten.
   - IR (CHCl₃):: 3615, 2930, 1730, 1570, 1452, 1390, 1040, 875 cm⁻¹.

### BEISPIEL 18

### (5RS)-5-Hydroxy-5-{3-[(1E,3E)-(5RS)-5-hydroxy-1,3-tridecadienyl]-phenyl}-pentansäure

Zu einer Lösung von 500 mg (5RS)-5-Acetoxy-5-{3-[(1E,3E)-(5RS)-5-Acetoxy-1,3-tridecadienyl]-phenyl}-pentan-1-ol-tert.-butyldimethylsilylether in 28 ml Tetrahydrofuran fügt man bei 0°C 914 mg Tetrabutylammoniumfluorid, rührt 30 Minuten bei 0°C und 4,5 Stunden bei 24°C. Anschließend wird mit Ether verdünnt, mit Sole gewaschen, über Natriumsulfat getrocknet und im Vakuum eingedampft. Der Rückstand wird an Kieselgel chromatographiert. Mit Hexan/0-20% Essigester erhält man 193 mg (5RS)-5-Acetoxy-5-{3-{(1E, 3E)-(5RS)-5-acetoxy-1,3-tridecadieny}-phenyl}-pentan-1-ol als farbloses Öl.
- IR (CHCl₃):: 3620, 3480, 2960, 2930, 2860, 1730, 1372, 1245, 1022, 990 cm⁻¹.

Zu einer Mischung von 1,25 g Collins-Reagenz (Chromsäure-Pyridin-Komplex) in 20 ml Methylenchlorid tropft man bei 0°C unter Rühren eine Lösung von 193 mg des vorstehend hergestellten Alkohols in 5 ml Methylenchlorid und rührt 1 Stunde bei 0°C. Anschließend wird so viel Celite hinzugegeben bis ein dicker Brei entsteht, dieser wird mit Hexan/Essigester (1:1) aufgeschlämmt, abfiltriert, gut mit Hexan/Essigester (1:1) gewaschen und das Filtrat im Vakuum eingedampft. Man erhält 150 mg (5RS)-5-Acetoxy-5-{3-[(1E,3E)-(5RS)-5-acetoxy-1,3-tridecadienyl]-phenyl}-pentanal als farbloses Öl.
- IR (CHCl₃):: 3020, 2960, 2930, 2860, 2730,1730, 1375, 1245, 1022, 990 cm⁻¹.

Zu einer Losung von 150 mg des vorstehend hergestellten Aldehyds in 11 ml Aceton tropft man unter Rühren bei -30°C 0,3 ml Jones Reagenz (J. Chem. Soc. 1953, 2555) und rührt eine Stunde bei -30°C. Anschließend fügt man 0,23 ml Isopropanol zu, rührt 15 Minuten, verdünnt mit Ether, filtriert, wäscht mit Sole neutral, trocknet über Magnesiumsulfat und dampft im Vakuum ein. Den Rückstand chromatographiert man an Kieselgel. Mit Hexan/0-50% Essigester erholt man 52 mg (5RS)-5-Acetoxy-5-{3-[(1E,3E)-(5RS)-5-acetoxy-1,3-tridecadienyl]-phenyl}-pentan-säure als farbloses Öl.
- IR (CHCl₃):: 3520, 3200, 2960, 2930, 2860, 1730, 1372, 1245, 1020, 990 cm⁻¹.

Zu einer Lösung von 34 mg der vorstehend hergestellten Säure in 0,62 ml Methanol gibt man bei 0°C 0,77 ml 0,5 n Natronlauge und rührt 1,8 Stunden bei 24°C. Anschließend wird mit 2,5 ml Wasser verdünnt und bei 0°C mit 0,5 n Schwefelsäure auf pH 6 angesäuert. Man extrahiert dreimal mit Essigester, schüttelt die organische Phase mit wenig Wasser, trocknet über Natriumsulfat und dampft im Vakuum ein. Der Rückstand wird an Kieselgel chromatographiert. Mit Hexan/30-85% Essigester erhält man 15 mg der Titelverbindung als farbloses Öl.
- IR (Film):: 3420, 2960, 2930, 2860, 1723, 1380, 1070, 990 cm⁻¹.

### Das Ausgangsmaterial für die obige Titelverbindung wird wie folgt hergestellt:

A. 5-[3-(tert.-Butyl-dimethylsilyloxymethyl)-phenyl]-(2E,4E)-pentadiensaureethylester
   Zu einer Losung von 30 g 3-Hydroxymethylbenzylalkohol und 30 g Imidazol in 250 ml Dimethylformamid fügt man bei 0°C unter Argon 33,3 g tert.-Butyldimethylsilylchlorid und rührt 20 Stunden bei 25°C. Man verdünnt mit 1,5 l Ether, schüttelt zweimal mit je 100 ml 10%iger Schwefelsäure, wäscht mit Sole neutral, trocknet über Natriumsulfat und dampft im Vakuum ein. Der Rückstand wird an Kieselgel chromatographiert. Mit Hexan/0-80% Ether erhält man 23,4 g 3-tert.-Butyldimethylsilyloxymethyl-benzylalkohol als farbloses Öl.
   - IR (CHCl₃):: 3680, 3610, 3440, 2960, 2930, 2860, 1470, 1255, 840 cm⁻¹.

   Eine Losung aus 21 g des vorstehend hergestellten Silylethers in 320 ml Methylenchlorid versetzt man mit 82 g Braunstein und rührt 8 Stunden bei 25°C. Anschließend wird filtriert und eingedampft. Man erhalt 20,7 g 3-tert.-Butyldimethylsilyloxymethyl-benzaldehyd als farbloses Öl.
   - IR (CHCl₃):: 2960, 2935, 2860, 1700, 1610, 1590, 1470, 1255, 840 cm⁻¹.

   Zur Wittig-Horner-Olefinierung fügt man bei -20°C 14 g Kalium-tert.-butylat zu einer Lösung aus 33,4 g Phosphonocrotonsäuretriethylester in 340 ml Tetrahydrofuran und rührt 30 Minuten bei -20°C. Anschließend tropft man in diese Lösung eine Lösung aus 20 g 3-tert.-Butyldimethylsilyloxymethylbenzaldehyd in 180 ml Tetrahydrofuran und rührt 1 Stunde bei -20°C. Man gießt dann auf 700 ml Eiswasser, extrahiert dreimal mit mit je 500 ml Ether,wäscht die organische Phase mit Sole neutral. trocknet über Natriumsulfat und dampft im Vakuum ein. Der so erhaltene Rückstand wird an Kieselgel chromatographiert. Mit Hexan/0-30% Ether erhält man 25,8 g der Titelverbindung als farbloses Öl.
   - IR (CHCl₃):: 2960, 2935, 2860, 1705, 1627, 1470, 1370, 1245, 998, 840 cm⁻¹.
B. 5-[3-(tert.-Butyl-dimethylsilyloxymethyl)-phenyl]-(2E,4E)-pentadienal
   Zu einer Lösung von 10,6 g des nach Beispiel 1A hergestellten Esters in 250 ml Toluol tropft man bei -70°C unter Argon 56 ml einer ca. 1,2 molaren Lösung von Diisobutylaluminiumhydrid in Toluol und rührt eine Stunde bei -70°C. Anschließend tropft man 10 ml Isopropanol gefolgt von 25 ml Wasser zu und rührt 2 Stunden bei 22°C, filtriert, wäscht mit Toluol und dampft im Vakuum ein. Man erhalt 10,6 g 5-[3-(tert.-Butyl-dimethylsilyloxymethyl)-phenyl]-(2E,4E)-pentadien-1-ol als farbloses Öl.
   - IR (CHCl₃):: 3610, 3450,3005, 2960, 2935, 2860, 1470, 1380, 1255, 990, 840 cm⁻¹.

   Eine Lösung von 10,6 g des vorstehend hergestellten Alkohols in 600 ml Methylenchlorid versetzt man mit 38 g Braunstein und rührt 4 Stunden bei 25°C. Anschließend wird filtriert und eingedampft. Man erhält 7,8 g der Titelverbindung als farbloses Öl.
   - IR(CHCl₃):: 3605, 2960, 2935, 2860, 1675, 1622, 1470, 1245, 988, 840 cm⁻¹.
C. 5-Acetoxy-1-[3-(tert.-butyl-dimethylsilyloxymethyl)-phenyl]-(1E,3E)-tridecadien
   Zu 1,12 g Magnesium in 5 ml Ether tropft man unter Erwärmen eine Lösung aus 7,96 ml n-Octylbromid in 12 ml Ether und rührt 30 Minuten bei 25°C. Zu 17 ml dieser Grignard-Lösung tropft man bei -20°C unter Argon eine Lösung aus 7,8 g des nach B hergestellten Aldehyds in 150 ml Ether und rührt 2 Stunden bei -20°C. Man versetzt mit 100 ml gesättigter Ammoniumchlorid-Lösung, extrahiert dreimal mit Ether, schüttelt die organische Phase mit Sole, trocknet über Magnesiumsulfat und dampft im Vakuum ein. Man erhält 11g 5-Hydroxy-1-[3-(tert.-butyl-dimethylsilyloxymethyl)-phenyl]-(1E,3E)-tridecadien als farbloses Öl.
   - IR (CHCl₃):: 3610, 3460, 3000,2960, 2930, 2860, 1470, 1255, 988, 840 cm⁻¹.

   Zur Acetylierung fügt man zu einer Lösung von 11g des vorstehend hergestellten Alkohols in 120 ml Pyridin 31 ml Essigsäureanhydrid und rührt 15 Stunden bei 23°C. Anschließend engt man im Vakuum unter Zusatz von Toluol ein und chromatographiert den Rückstand an Kieselgel. Mit Hexan/0-3% Essigester erhält man 8,9 g der Titelverbindung als farbloses Öl.
   - IR (CHCl₃):: 2960, 2937, 2860,1730,1470, 1372, 1255, 990, 840 cm⁻¹.
D. (5RS)-5-Acetoxy-5-{3-[(1E,3E)-(5RS)-5-acetoxy-1,3-tridecadienyl]-phenyl}-pentan-1-ol-tert.-butyldimethylsilylether
   Zu einer Lösung von 2,4 g des nach C hergestellten Acetats in 150 ml Tetrahydrofuran fügt man bei 0°C 4,9 g Tetrabutylammoniumfluorid und rührt 1,5 Stunden bei 24°C. Anschließend verdünnt man mit 250 ml Ether, schüttelt mit Sole, trocknet über Natriumsulfat und dampft im Vakuum ein. Der erhaltene Rückstand wird an Kieselgel chromatographiert. Mit Hexan/0-25% Essigester erhält man 1,5 g 3-[(1E,3E)5-acetoxy-1,3-tridecadienyl]-benzylalkohol als farbloses Öl.
   - IR (CHCl₃):: 3610, 3440, 3005, 2960, 2930, 2860, 1730, 1375, 1250, 990 cm⁻¹.

   Zu einer Lösung von 1,5 g des vorstehend hergestellten Benzylalkohols in 30 ml Methylenchlorid fügt man 11 g Braunstein und rührt 3 Stunden bei 24°C. Anschließend wird filtriert, mit Methylenchlorid gewaschen und im Vakuum eingedampft. Man erhält 1,3 g 3-[(1E,3E)-5-acetoxy-1,3-tridecadienyl]-benzaldehyd als farbloses Öl.
   - IR (CHCl₃):: 3020, 2960, 2930, 2860, 2740, 1727, 1695, 1600, 1372, 1245, 988 cm⁻¹.

   Zu 1,4 g Magnesium tropft man bei 25°C unter Argon eine Lösung aus 6,7 g 4-Chlor-1-tert.-butyldimethylsilyloxybutan in 6 ml Tetrahydrofuran und 0,187 ml Dibromethan, erwärmt 5 Minuten auf 70°C, rührt 30 Minuten bei 25°C und verdünnt mit 18,8 ml Tetrahydrofuran.
   Zu 16 ml dieser magnesiumorganischen Lösung tropft man bei -70°C unter Argon eine Losung aus 1,3 g 3-[(1E,3E)-5-acetoxy-1,3-tridecadienyl]-benzaldehyd in 16 ml Tetrahydrofuran und rührt 1,5 Stunden bei -70°C. Man versetzt mit 150 ml gesättigter Ammoniumchlorid-Lösung, extrahiert 3 mal mit Ether, schüttelt die organische Phase mit Sole, trocknet über Magnesiumsulfat und dampft im Vakuum ein. Der Rückstand wird in 22 ml Pyridin gelöst, mit 12 ml Essigsäureanhydrid versetzt und 16 Stunden bei 22°C gerührt. Anschließend engt man im Vakuum unter Zusatz von Toluol ein und chromatographiert den Rückstand an Kieselgel (Flash-Chromatographie). Mit Hexan/0-3,5% Essigester erhält man 1,1 g der Titelverbindung als farbloses Öl.
   - IR (CHCl₃):: 3005, 2960, 2930, 2860,1730,1372, 1250, 1095, 988, 838 cm⁻¹.

### BEISPIEL 19

### (5RS)-5-Hydroxy-5-{3-[(1E)-(3RS)-3-hydroxy-1-undecenyl]-phenyl}-pentansäure

Zu einer Lösung von 630 mg (5RS)-5-Acetoxy-5-{3-[(1E)-(3RS)-3-Diphenyltert.-butylsilyloxy-1-undecenyl]-phenyl}-pentan-1-ol-tert.-butyldimethylsilylether in 12 ml Ethanol gibt man bei 22°C unter Argon 630 mg Pyridinium-p-toluolsulfonat und rührt 3,5 Stunden bei 22°C. Anschließend verdünnt man mit Ether, wäscht mit Wasser, trocknet über Natriumsulfat und dampft im Vakuum ein. Der Rückstand wird an Kieselgel chromatographiert. Mit Hexan/0-20% Essigester erhält man 472 mg (5RS)-5-Acetoxy-5-{3-[(1E)-(3RS)-3-diphenyl-tert.-butylsilyloxy-1-undecenyl]-phenyl}-pentan-1-ol als farbloses Öl.
- IR (CHCl₃):: 3620, 3450, 3000, 2960, 2930, 2860, 1730, 1375, 1245, 1110, 965, 700 cm⁻¹.

Zu einer Lösung von 470 mg des vorstehend hergestellten Alkohols in 50 ml Methylenchlorid gibt man bei 0°C unter Argon 4 g Collins-Reagenz (Chromsäure-Pyridin-Komplex) und rührt 30 Minuten bei 0°C. Anschließend wird soviel Celite hinzugegeben bis ein dicker Brei entsteht. Dieser wird mit Hexan/Ether (1+1) aufgeschlämmt, abfiltriert, gut mit Hexan/Ether (1+1) gewaschen und das Filtrat im Vakuum eingedampft. Man erhält 456 mg (5RS)-5-Acetoxy-5-{3-[(1E)-(3RS)-3-Diphenyl-tert.butylsilyloxy-1-undeccenyl]-phenyl}-pentanal als farbloses Öl.
- IR (CHCl₃):: 3000, 2960, 2930, 2860, 2730, 1728, 1372, 1245, 1110, 968, 700 cm⁻¹.

Zu einer Lösung von 456 mg des vorstehend hergestellten Aldehyds in 20 ml Aceton tropft man unter Rühren bei -30°C 1 ml Jones-Reagenz (J.Chem.Soc. 1953, 2555) und rührt 35 Minuten bei -20°C. Anschließend fügt man 0,3 ml Isopropanol zu, rührt 10 Minuten bei -20°C, verdünnt mit Ether, filtriert, wäscht mit Sole neutral, trocknet über Natriumsulfat und dampft im Vakuum ein. Den Rückstand chromatographiert man an Kieselgel. Mit Hexan/0-50% Essigester erhält man 397 mg (5RS)-5-Acetoxy-5-{3-[(1E)-(3RS)-3-diphenyl-tert.-butylsilyloxy-1-undecenyl]-phenyl}-pentansäure als farbloses Öl.
- IR (CHCl₃):: 3520, 3180, 2950, 2930, 2860, 1730, 1375, 1245, 1110, 968, 700 cm⁻¹.

Zu einer Lösung von 390 mg der vorstehend hergestellten Säure in 20 ml Tetrahydrofuran fügt man unter Argon bei 24°C 390 mg Tetrabutylammoniumfluorid und rührt 6 Stunden bei 24°C. Anschließend verdünnt man mit Ether, schüttelt die organische Phase mit Sole, trocknet über Natriumsulfat und dampft im Vakuum ein. Der Rückstand wird an Kieselgel chromatographiert. Mit Hexan/10-70% Essigester erhält man 278 mg (5RS)-5-Acetoxy-5-{3-[(1E)-(3RS)-3-hydroxy-1-undecenyl]-phenyl}-pentansäure als farbloses Öl.
- IR (CHCl₃):: 3610, 3520, 3200, 3010, 2960, 2930, 2860, 1730, 1375, 1245, 970 cm⁻¹.

Zu einer Lösung von 90 mg der vorstehend hergestellten Säure gibt man bei 22°C 0,5 n Natronlauge und rührt 2 Stunden bei dieser Temperatur. Anschließend wird mit 5 ml Wasser verdünnt und bei 0°C mit 10%iger Schwefelsäure auf pH 5 angesäuert. Man extrahiert viermal mit Essigester, schüttelt die organische Phase mit Sole, trocknet über Nritriumsulfat und dampft im Vakuum ein. Den Rückstand chromatographiert man an Kieselgel. Mit Hexan/30-90% Essigester erhält man 61 mg der Titelverbindung als farbloses Öl.
- IR (CHCl₃):: 3610, 3410, 3160, 3005, 2960, 2930, 2860, 1730, 1240, 968 cm⁻¹.

Das Ausgangsmaterial für die obige Titelverbindung wird wie folgt hergestellt:
A. 3-Diphenyl-tert.butylsilyloxy-1-[3-(tert.-butyl-dimethylsilyloxymethyl)-phenyl]-(1E)-undecen
   Zur Wittig-Horner-Olefinierung fügt man bei -20°C 3,64 g Kalium-tert.-butylat zu einer Lösung aus 9,41 g 2-Oxo-decyl-phosphonsäuredimethylester in 280 ml Tetrahydrofuran und rührt 20 Minuten bei dieser Temperatur. Anschließend tropft man in diese Lösung eine Lösung aus 4,5 g 3-tert.-Butyldimethylsilyloxymethylbenzaldehyd (s. Beispiel 18A) in 150 ml Tetrahydrofuran und rührt eine Stunde bei -20°C. Man gießt auf 500 ml gesättigte Ammoniumchlorid-Lösung, extrahiert mit Essigester, wäscht die organische Phase mit Sole neutral, trocknet über Natriumsulfat und dampft im Vakuum ein. Der so erhaltene Rückstand wird an Kieselgel chromatographiert. Mit Hexan/5-20% Essigester erhält man 6,5 g 1-[3-(Tert.-butyl-dimethylsilyloxymethyl)-phenyl]-(1E undecen-3-on als farbloses Öl.
   - IR (CHCl₃):: 3000, 2960, 2930, 1690, 1655, 1612, 1470, 1258, 970, 840 cm⁻¹.

   Zu einer Lösung von 6,5 g des vorstehend hergestellten Ketons in 270 ml Methanol gibt man unter Argon bei 0°C 483 mg Natriumborhydrid hinzu und rührt eine Stunde bei dieser Temperatur. Anschließend versetzt man mit 1,5 ml Eisessig, verdünnt mit 150 ml Wasser und extrahiert dreimal mit je 200 ml Essigester. Die organische Phase wird mit Sole neutral gewaschen, über Magnesiumsulfat getrocknet und im Vakuum eingedampft. Der Rückstand wird an Kieselgel chromatographiert. Mit Hexan/0-20% Essigester erhält man 5,65 g 1-[3-(Tert.-butyl-dimethylsilyloxymethyl)-phenyl]-(1E)-undecen-3-ol als farbloses Öl.
   - IR (CHCl₃):: 3610, 3000, 2960, 2930, 2860, 1470, 1255, 968, 840 cm⁻¹.

   Zu einer Lösung von 5,65 g des vorstehend hergestellten Alkohols in 100 ml Dimethylformamid fügt man bei 0°C unter Argon 3,6 g Imidazol und 6,18 g tert.-Butyldiphenylsilylchlorid und rührt 16 Stunden bei 22°C. Anschließend gibt man auf Wasser, extrahiert mit Hexan/Ether (1+1) , wäscht die organische Phase mit Wasser, trocknet über Natriumsulfat und dampft im Vakuum ein. Der Rückstand wird an Kieselgel chromatographiert. Mit Hexan/0-10% Essigester erhält man 5,1 g der Titelverbindung als farbloses Öl.
   - IR (CHCl₃):: 3005, 2960, 2930, 2860, 1470, 1255, 970, 840, 700 cm⁻¹.
B. (5RS)-5-{3-[(1E)-(3RS)-3-Diphenyl-tert.-butylsilyloxy-1-undecenyl]-phenyl}-5-acetoxy-pentan-1-ol-tert.-butyldimethylsilylether
   Zu einer Losung des nach Beispiel 19A hergestellten Silylethers in 100 ml Tetrahydrofuran gibt man 50 ml eines Gemisches aus Essigsäure/Wasser/Tetrahydrofuran (65+35+10) rührt 8 Stunden bei 50°C und 16 Stunden bei 24°C. Anschließend dampft man unter Toluolzusatz ein und erhalt 5,1 g 3-[(1E)-3-Diphenyl-tert.-butylsilyloxy-1-undecenyl]-benzylalkohol als farblose Öl.
   - IR (CHCl₃):: 3610, 3470, 3000, 2960, 2930, 2860, 1470, 1250, 970 700 cm⁻¹.

   Zu einer Lösung von 1,5 g des vorstehend hergestellten Alkohols in 20 ml Methylenchlorid fügt man 6 g Braunstein und rührt 4 Stunden bei 22°C. Anschließend wird über Celite filtriert, mit Methylenchlorid gewaschen und im Vakuum eingedampft. Der Rückstand wird an Kieselgel chromatographiert. Mit Hexan/Essigester (9+1) erhält man 1,47 g 3-[(1E)-3-Diphenyl-tert.-butylsilyloxy-1-undecenyl]-benzaldehyd als farbloses Öl.
   - IR (CHCl₃):: 3000, 2960, 2930, 2860, 2740, 1700, 1605, 1112, 970, 705 cm⁻¹.

   Zu einer Lösung von 1,47 g des vorstehend hergestellten Aldehyds in 10 ml Tetrahydrofuran tropft man unter Argon bei -70°C 20 ml einer Grignard-Lösung aus 4-Chlor-1-tert.-butyldimethylsilyloxybutan und Magnesium in Tetrahydrofuran (s. Beispiel 18D) und rührt 30 Minuten. Man gibt auf gesättigte Ammoniumchlorid-Lösung, extrahiert mit Ether, wäscht die organische Phase mit Wasser, trocknet über Natriumsulfat und dampft im Vakuum ein. Der Rückstand wird in 8 ml Pyridin gelöst, mit 2 ml Essigsäureanhydrid versetzt und 20 Stunden bei 24°C gerührt. Anschließend engt man im Vakuum unter Zusatz von Toluol ein und chromatographiert den Rückstand an Kieselgel. Mit Hexan/0-15% Essigester erhält man 630 mg der Titelverbindung als farbloses Öl.
   - IR (CHCl₃):: 3000, 2965, 2940, 2855, 1730, 1470, 1375, 1250, 1105, 968, 840, 700 cm⁻¹.

### BEISPIEL 20

### 5-{6-[(1E)-(3RS)-3-Hydroxy-1-undecenyl]-2-pyridyl}-4-oxo-pentansäuremethylester

A. Eine Lösung von 4,73 g 2,6-Dibrompyridin in 150 ml Triethylamin wird unter Eiskühlung mit 2,69 g Pentinsäuremethylester, 350 mg Bis-triphenylphosphinpalladium-II-chlorid und 48 mg Kupfer-I-jodid versetzt und die Mischung 1 Stunde gerührt. Danach wird das Eisbad entfernt und 3 Stunden bei Raumtemperatur gerührt. Der Niederschlag - bestehend aus 2,6-Bis-(4-methoxycarbonyl-1-butinyl)-pyridin und Triethylammoniumbromid - wird abgesaugt und das Filtrat zur Trockne eingeengt. Der Rückstand wird an Kieselgel mit Hexan/Ethylacetat = 95/5 bis 75/25 chromatographiert. Es werden 2,32 g 5-(6-Brom-2-pyridyl)-4-pentinsäuremethylester vom Schmelzpunkt 59-61°C erhalten.
   - IR(KBr):: 3040, 2230, 1728, 1572, 1548, 1433, 1162, 800 cm⁻¹.
B. Eine Mischung aus 500 mg rotem Quecksilberoxid, 0,2 ml Bortrifluorid-Diethylether-Komplex und 10 mg Trichloressigäsure in 1 ml Methanol wird auf 60° erwärmt und zu dieser Katalysatorlösung bei Raumtemperatur eine Losung von 9,4 g 5-(6-Brom-2-pyridyl)-4-pentinsäuremethylester in 15 ml Methanol gegeben und die Mischung 2 Stunden bei Raumtemperatur gerührt. Danach wird die Reaktionsmischung in 5%ige Schwefelsäure gegossen, mit Ethylacetat extrahiert, getrocknet (Na₂SO₄) und eingeengt. Es werden 6,3 g 5-(6-Brom-2pyridyl)-4-oxo-pentansäuremethylester als hellgelbes Öl erhalten.
   - IR:: 2955, 1738, 1720, 1585, 1555, 1440, 1410, 1360, 1200, 1165, 1120, 988 cm⁻¹.
C. Unter den Bedingungen des Beispiels 5B werden 1,3 g 5-(6-Brom-2-pyridyl)-4-oxo-pentansäuremethylester und 2,31 g (1E)-1-Tri-n-butylstannyl)-1-undecen-(3RS)-3-ol in 13 ml Dimethylformamid in Gegenwart von 320 mg 1,1'-Bis(diphenylphosphino)-ferrocenpalladium-II-chlorid als Katalysator umgesetzt, und analog Beispiel 11B aufbereitet. Das Rohprodukt wird an Kieselgel mit Hexan/Ethylacetat 95/5 bis 75/25 chromatographiert. Es werden 300 mg der Titelverbindung als hellgelbes Öl erhalten.
   - IR (CHCl₃):: 3610, 2925, 1738, 1725, 1573, 1453, 1260, 1040 cm⁻¹.

### BEISPIEL 21

### (4RS)-4-Hydroxy-5-{6-[(1E)-(3RS)-3-hydroxy-1-undecenyl]-pyridyl}-pentansäure

A. Eine Lösung von 490 mg 5-(6-Brom-2-pyridyl)-4-oxo-pentansäuremethylester in 20 ml Methanol wird unter Eiskühlung mit 72 mg Natriumborhydrid versetzt und 1 Stunde bei 0-3° C gerührt. Danach werden 2 ml Aceton hinzugefügt, 1 Stunde nachgerührt und die Reaktionsmischung zur Trockne eingeengt. Der Rückstand wird Zwischen Wasser und Ethylacetat verteilt, die Organische Phase über Natriumsulfat getrocknet und eingeengt. Es werden 421 mg 5-[(6-Brom-2-pyridyl)-methyl]-tetrahydrofuran-2-on als gelbes Öl erhalten.
   - IR (CHCl₃):: 1765, 1583, 1553, 1423, 1170, 1160, 1118, 1020, 985 cm⁻¹.
B. Unter der Bedingungen des Beispiels 11 B werden 400 mg 5-[(6-Brom-2-pyridyl)-methyl]-tetrahydrofuran-2-on und 780 mg (1E)-1-(Tri-n-butylstannyl)-1-undecen-(3RS)-3-ol in 5 ml Dimethylformamid in Gegenwart von 112 mg 1,1'-Bis-(diphenylphosphino)-ferrocen-palladium-II-chlorid als Katalysator umgesetzt und aufbereitet. Das Rohprodukt wird an Kieselgel mit Hexan/0-30 % Ethylacetat chromatographiert. Es werden 146 mg 5-{6-[(1E)-(3RS)-3-Hydroxy-1-undecenyl]-2-pyridyl-methyl}-tetrahydrofuran-2-on als gelbes Öl erhalten.
   - IR (CHCl₃):: 2915, 2840, 1762, 1667, 1585, 1570, 1450, 1350, 1170, 970 cm⁻¹.
C. Unter den Bedingungen dem Beispiels 2 werden 30 mg 5-{6-[(1E)-(3RS)-3-Hydroxy-1-undecenyl]-2-pyridyl-methyl}-tetrahydrofuran-2-on in 2 ml Methanol mit 1 ml 1 n Natronlauge verseift und aufbereitet. Es werden 20 mg der Titelverbindung als Öl erhalten.
   - IR (CHCl₃):: 3550-3080, 2920, 1724, 1585, 1570, 1450, 1255, 1090, cm⁻¹.

### BEISPIEL 22

### 5-{6-(1E)-(3RS)-3-Hydroxy-1-undecenyl]-2-pyridyl}-(4RS)-4-hydroxypentan-1-ol

A. Eine Lösung von 500 mg 5-[(6-Brom-2-pyridyl)-methyl]-tetrahydrofuran-2-on in 13 ml Triethylamin und 2 ml Tetrahydrofuran wird mit 350 mg (3RS)-1-Undecin-3-ol, 37 mg Bis-(triphenylphosphin)-palladium-II-chlorid und 5 mg Kupfer-I-iodid versetzt und die Mischung 3 Tage bei Raumtemperatur gerührt. Die Reaktionsmischung wird zur Trockne eingeengt, zwischen Wasser und Ethylacetat verteilt, über Natriumsulfat getrocknet, eingeengt und der Rückstand an Kieselgel mit Hexan/0-35% Ethylacetat chromatographiert. Es werden 294 mg 5-{6-[(3RS)-3-Hydroxy-1-undecinyl]-2-pyridyl-methyl}-tetrahydrofuran-2-on als Öl erhalten.
   - IR (CHCl₃):: 2920, 2850, 2220, 1768, 1585, 1450, 1173 cm⁻¹.

   Die Darstellung von (3RS)-1-Undecin-3-ol ist in der deutschen Patentanmeldung P 39 09 326.3 beschrieben.
B. Zu einer Lösung von 0,725 ml Natrium-bis-(2-methoxyethoxy)-aluminiumhydrid (3,5 molar in Toluol), die mit 3,75 ml Toluol verdünnt wurde, wird unter Argonatmosphäre bei 0° C eine Lösung von 145 mg 5-{6-[(3RS)-3-Hydroxy-1-undecinyl]-2-pyridyl-methyl}-tetrahydrofuran-2-on in 1,2 ml Toluol getropft. Nach beendeter Zugabe wird die Reaktionsmischung bei Raumtemperatur gerührt und nach 3 Stunden unter Eiskühlung nacheinander mit 0,5 ml Isopropanol und 0,5 ml Wasser versetzt.
   Der Niederschlag wird abgesaugt mit Ethylacetat gewaschen, das Filtrat eingeengt und der Rückstand an Kieselgel mit Methylenchlorid/0-5 % Methanol chromatographiert. Es werden 22 mg der Titelverbindung als gelbes Öl erhalten
   - IR (CHCl₃):: 3550-3080, 2920, 2859, 1585, 1570, 1450, 1255, 1180 cm⁻¹.

### BEISPIEL 23

### (5RS)-5-Hydroxy-5-{6-[(1E)-(3RS)-3-hydroxy-1-undecenyl]-2-pyridyl}-pentan-1-ol

A. Eine Lösung von 4 g 2-Brom-6-[(1E)-(3RS)-3-hydroxy-1-undecenyl]-pyridin in 20 ml Dimethylformamid wird mit 4,1 g tert.-Butyl-diphenylsilylchlorid und 2,1 g Imidazol versetzt und 15 Stunden bei Raumtemperatur gerührt. Die Reaktionsmischung wird in 100 ml Diethylether gegossen, 2 mal mit 20 ml 1 n Salzsäure, 4 mal mit 20 ml gesättigter Kochsalzlösung ausgeschüttelt, über Natriumsulfat getrocknet und eingeengt. Der Rückstand wird an Kieselgel mit Hexan/0-5 % Ethylacetat chromatographiert. Es werden 5,5 g 2-Brom-6-[(1E)-(3RS)-3-tert.-butyldiphenylsilyloxy-1-undecenyl]-pyridin als Öl erhalten.
   - IR (CHCl₃):: 2925, 2850, 1578, 1548, 1426, 1110, 983, 820, 695 cm⁻¹.
B. Zu einer Suspension von 500 mg 2-Brom-6-[(1E)-(3RS)-3-tert.-butyl-diphensilyloxy-1-undecenyl]-pyridin in 2 ml Diethylether und 1 ml Tetrahydrofuran werden bei -80° unter Argonatmosphäre 1,16 ml n-Butyllithium (1,6 molar in Hexan) getropft. Nach beendeter Zugabe wird die Reaktionsmischung 15 Minuten bei -40° C gerührt auf -80° C gekühlt und tropfenweise bei dieser Temperatur mit 140 mg Dimethylformamid versetzt. Nach 4 Stunden bei -80° C wird die Reaktionsmischung mit 3 ml 2 n Salzsaure hydrolysiert, die organische Phase abgetrennt, über Natriumsulfat getrocknet, eingeengt und der Rückstand an Kieselgel mit Hexan/0-10 % Ethylacetat chromatographiert. Es werden 385 mg 6-[(1E)-(3RS)-3-tert.-Butyl-diphenylsilyloxy-1-undecenyl]-pyridin-2-carbaldehyd als gelbes Öl erhalten.
   - IR (CHCl₃):: 2915, 2845, 1705, 1768, 1580, 1450, 1420, 1105, 695 cm⁻¹.
C. 5,1 ml einer Grignardlösung (aus 385 mg Magnesium und 1,76 g 4-Chlor-1-tert.-butyldimethylsilyloxybutan in Tetrahydrofuran hergestellt) werden bei -80° C zu einer Lösung von 370 mg 6-[(1E)-(3RS)-3-tert.-Butyl-diphenylsilyloxy-1-undecenyl]-pyridin-2-carbaldehyd in 3 ml Tetrahydrofuran getropft. Nach 2-stündigem Rühren bei -80° C wird die Reaktionsmischung in 10 ml gesättigte Ammoniumchlorid-Lösung gegossen, mit Ethylacetat ausgeschüttelt, die organische Phase getrocknet und eingeengt. Der Rückstand wird in 10 ml Tetrahydrofuran gelöst, mit 2,47 g Tetrabutylammoniumfluorid versetzt und 17 Stunden bei Raumtemperatur gerührt. Die Reaktionsmischung wird in 50 ml Diethylether gegossen, mit gesättigter Kochsalzlösung gewaschen, über Natriumsulfat getrocknet und eingeengt. Der Rückstand wird an Kieselgel mit Hexan/0-30 % Ethylacetat chromatographiert. Es werden 110 mg der Titelverbindung als gelbes Ol erhalten.
   - IR (CHCl₃):: 2920, 2850, 1590, 1570, 1455, 1258, 1075, 1015, 970 cm⁻¹.

### BEISPIEL 24

### 2-[(2RS)-2-Hydroxy-2-(3-methoxycarbonylphenyl)-ethyl]-6-[(1E)-(3RS)-3-hydroxy-1-undecenyl]-pyridin

A. Eine Lösung von 15,1 g 2,6-Dibrompyridin in 500 ml Triethylamin wird unter Eiskühlung mit 10,2 g 3-Ethinylbenzoesäuremethylester, 1,1 g Bis-(triphenylphosphin)-palladium-II-chlorid und 150 mg Kupfer-I-iodid versetzt und die Mischung 1 Stunde gerührt. Danach wird das Eisbad entfernt und 48 Stunden bei Raumtemperatur gerührt. Der Niederschlag wird abfiltriert, das Filtrat zur Trockne eingeengt, mit Wasser versetzt und mit Ethylacetat ausgeschüttelt. Die organische Phase wird über Natrtiumsulfat getrocknet, eingeengt und der Rückstand an Kieselgel mit Hexan/0-12 % Ethylacetat chromatographiert. Es werden 4,8 g 2-Brom-6-[2-(3-methoxycarbonylphenyl)-ethinyl]-pyridin vom Schmelzpunkt 124-126° C erhalten.
   - IR (CHCl₃):: 2980, 2210, 1715, 1570, 1430, 1280, 1260, 1165, 1103 cm⁻¹.
B. Eine Lösung von 5,2 g 2-Brom-6-[2-(3-methoxycarbonylphenyl)-ethinyl]-pyridin in 30 ml 60 %iger Schwefelsäure wird unter Rühren 1,5 Stunden auf 140° C erhitzt. Nach dem Abkühlen auf Raumtemperatur wird die Reaktionsmischung auf Eiswasser gegossen, der Niederschlag abgesaugt und mit Wasser neutral gewaschen. Nach dem Trocknen bei 50° C im Vakuum wird das Rohprodukt in 35 ml Methanol gelöst, mit 3 Tropfen konzentrierter Schwefelsäure versetzt und 8 Stunden am Rückfluß erhitzt. Danach wird die Reaktionsmischung eingeengt, zwischen Wasser und Ethylacetat verteilt, die organische Phase mit 10 %iger Natriumhydrogencarbonat und Wasser gewaschen über Natriumsulfat getrocknet und eingeengt. Es werden 3,5 g 2-Brom-6-[(3-methoxycarbonylbenzoyl)methyl]-pyridin vom Schmelzpunkt 84-86° C erhalten.
   - IR (CHCl₃):: 1720, 1633, 1590, 1438, 1300, 1165 cm⁻¹.
C. Eine Lösung von 2,4 g 2-Brom-6-[2-(3-methoxycarbonylbenzoyl)-methyl]-pyridin in 100 ml Methanol wird unter Eiskühlung mit 293 mg Natriumborhydrid versetzt, 1 Stunde bei 0° C und über Nacht bei Raumtemperatur geruhrt. Danach werden 3 ml Aceton hinzugefügt, eine halbe Stunde nachgerührt und die Reaktionsmischung zur Trockne eingeengt. Der Rückstand wird zwischen Wasser und Ethylacetat verteilt, die organische Phase mit Natriumsulfat getrocknet und eingeengt. Es werden 2,33 g 2-Brom-6-[(2RS)-2-hydroxy-2-(3-methoxycarbonylphenyl)-ethyl]pyridin als gelbes Öl erhalten.
   - IR (CHCl₃):: 3540-3280, 1715, 1585, 1550, 1434, 1285, 1104 cm⁻¹.
D. Eine Lösung vopn 120 mg 2-Brom-6-[(2RS)-2-hydroxy-2-(3-ethoxycarbonylphenyl)-ethyl]-pyridin in 0,7 ml Toluol wird mit 180 mg (1E)-1-(Tri-n-butylstannyl)-1-undecen-(3RS)-3-ol und 22 mg 1,1'-Bis-(diphenylphosphino)-ferrocen-palladium-II-chlorid versetzt und unter Argonatmosphäre 2 Stunden bei 100° C gerührt. Die Reaktionsmischung wird zur Trockne eingeengt und der Rückstand an Kieslgel mit Hexan/0-15 % Ethylacetat chromatographiert. Es werden 56 mg der Titelverbindung als Öl erhalten.
   - IR (CHCl₃):: 2920, 2850, 1715, 1500, 1450, 1285, 1260 1090, 1005 cm⁻¹.

### BEISPIEL 25

### 2-[(2RS)-2-Hydroxy-2-(3-carboxyphenyl)-ethyl]-6-[(1E)-(3RS)-3-hydroxy-1-undecenyl]-pyridin

Unter den Bedingungen des Beispiels 2 werden 30 mg 2-[(2RS)-2-Hydroxy-2-(3-methoxycarbonylphenyl)-ethyl]-6-[(1E)-(3RS)-3-hydroxy-1-undecenyl]-pyridin in 2 ml Methanol mit 1 ml 1 n Natronlauge verseift und aufbereitet. Es werden 18 mg der Titelverbindung als Öl erhalten.
¹H-NMR (300 MHz, CD₂Cl₂, δ ppm): 0,80 (t,J=6Hz,3H): 1,0-1,62 (14H): 2,95-3,17(2H); 4,21-4,32(1H); 5,08-5,20(1H); 6,57-6,72 (2H); 6,90-6,98(1H); 7,11-7,20(1H); 7,32-7,42 (1H); 7,50-7,65(2H); 7,82-7,93(1H); 8,02-8,13 (1H).

### BEISPIEL 26

### 2-[(2RS)-2-Hydroxy-2-(3-hydroxymethylphenyl)-ethyl]-6-[(1E)-(3RS)-3-hydroxy-1-undecenyl]-pyridin

A. Eine Lösung von 180 mg 2-Brom-6-[(2RS)-2-hydroxy-2-(3-methoxycarbonylphenyl)-ethyl]-pyridin in 4 ml Toluol wird unter Argonatmosphäre bei -70° C mit 0,88 ml Diisobutylaluminiumhydrid (20 %ig in Toluol) versetzt und 3 Stunden bei dieser Temperatur gerührt. Die Reaktionsmischung wird nacheinander bei -70° C mit 0,3 ml Isopropanol und 0,3 ml Wasser versetzt und über Nacht bei Raumtemperatur gerührt. Der Niederschlag wird abfiltriert, das Filtrat über Natriumsulfat getrocknet und eingeengt. Das Rohprodukt wird in 3 ml Methanol gelöst, mit 82 mg Natriumborhydrid versetzt und über Nacht bei Raumtemperatur gerührt. Die Reaktionsmischung wird mit 2 n Salzsäure auf pH 1 angesäuert, mit Ethylacetat ausgeschüttelt, die organische Phase über Natriumsulfat getrocknet, eingeengt und der Rückstand an Kieselgel mit Hexan/0-10 % Ethylacetat chromatographiert. Es werden 103 mg 2-Brom-6-[(2RS)-2-Hydroxy-2-(3-hydroxymethylphenyl)-ethyl]-pyridin als farbloses Öl erhalten.
   - IR (CHCl₃):: 3680-3080, 2910, 2860, 1580, 1548, 1430, 1400, 1150, 1113, 1040, 788 cm⁻¹.
B. Unter den Bedingungen des Beispiels 24 D werden 96 mg 2-Brom-6-[(2RS)-(2-Hydroxy-2-(3-hydroxymethylphenyl)-ethyl]-pyridin und 160 mg (1E)-1-(Tri-n-butyl-stannyl)-1-undecen-(3RS)-3-ol in 1 ml Toluol in Gegenwart von 1,1'-Bis-(diphenylphosphino)-ferrocen-palladium-II-chlorid als Katalysator umgesetzt und aufbereitet. Das Rohprodukt wird an Kieselgel mit mit Hexan/0-25 % Ethylacetat chromatographiert. Es werden 20 mg der Titelverbindung als weinrotes Öl erhalten.
   - IR (CHCl₃):: 2920, 2845, 1584, 1567, 1448, 1235, 1090, 1007 cm⁻¹.

### BEISPIEL 27

### 5-{6-[(1E)-(3RS)-3-Hydroxy-1-undecenyl]-2-pyridyl}-4-pentinsäure

A. Unter den Bedingungen des Beispiels 11 B werden 1,4 g 5-(6-Brom-2-pyridyl)-4-pentinsäuremethylester und 5,75 g (1E)-1-(Tri-n-butylstannyl)-1-undecen-(3RS)-3-ol in 30 ml Dimethylformamid in Gegenwart von 185 mg 1,1'-Bis-(diphenylphosphino)-ferrocen-palladium-II-chlorid als Katalysator umgesetzt und aufbereitet. Das Rohprodukt wird an Kieselgel mit Hexan/0-20 % Ethylacetat chromatographiert. Es werden 354 mg 5-{6-[(1E)-(3RS)-3-Hydroxy-1-undecenyl]-2-pyridyl}-4-pentinsäuremethylester als gelbes Öl erhalten.
   - IR (CHCl₃):: 3030, 2400, 1740, 1520, 1430, 1218, 1048, 930 cm⁻¹.
B. Unter den Bedingungen des Beispiels 2 werden 20 mg 5-{6-[(1E)-(3RS)-3-Hydroxy-1-undecenyl]-2-pyridyl}-4-pentinsäuremethylester in 1 ml Methanol mit 2 ml 1 n Natronlauge verseift und aufbereitet. Es werden 11 mg der Titelverbindung als gelbes Öl erhalten.
   - IR (CHCl₃):: 3690, 3040, 3020, 2930, 2860, 2400, 1730, 1605, 1260, 1100, 1015, 930 cm⁻¹.

## Patentansprüche

1. Leukotrien-B₄-Analoge der Formel I, 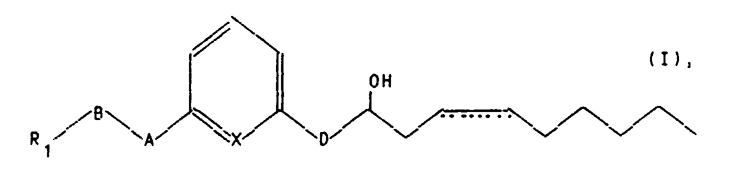 worin
R¹ den Rest COOR² mit R² in der Bedeutung eines Wasserstoffatoms oder einer (C₁-C₄)-Alkylgruppe oder R¹ den Rest CH₂OH,
B eine Alkylengruppe mit 1-3 C-Atomen in der Kette, einen Rest 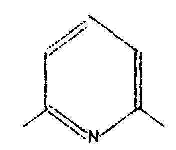 oder einen Rest 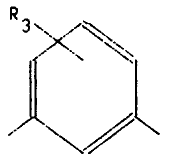 mit R³ in der Bedeutung eines Wasserstoffatoms, einer Carboxy- oder Methoxycarbonyl-Gruppe,
A die Gruppen 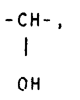 -O-, 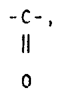 -NH-CO-, -CO-NH-, -OCH₂-, -CH=CH-, -C≡C-, -COCH₂-,
oder -CHOH-CH₂-,
X N oder CH,
D die Gruppen 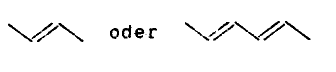 und
..... eine Einfach- oder Doppelbindung bedeuten, sowie gegebenenfalls deren Salze mit physiologisch unbedenklichen Basen.

2. Verfahren zur Herstellung von Leukotrien-B₄-Analoge der Formel I, 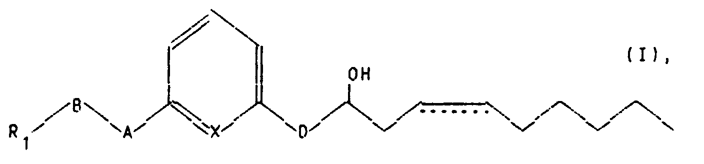 worin
R¹ den Rest COOR² mit R² in der Bedeutung eines Wasserstoffatoms oder einer (C₁-C₄)-Alkylgruppe oder R¹ den Rest CH₂OH,
B eine Alkylengruppe mit 1-3 C-Atomen in der Kette, einen Rest 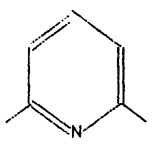 oder einen Rest 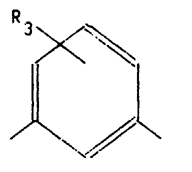 mit R³ in der Bedeutung eines Wasserstoffatoms, einer Carboxy- oder Methoxycarbonyl-Gruppe,
A die Gruppen 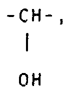 -O-, 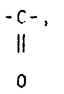 -NH-CO-, -CO-NH-, -OCH₂-, -CH=CH-, -C≡C-,
oder -CHOH-CH₂-,
X N oder CH,
D die Gruppen 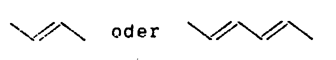 und
..... eine Einfach- oder Doppelbindung bedeuten, sowie gegebenenfalls deren Salze mit physiologisch unbedenklichen Basen, dadurch gekennzeichnet daß man in an sich bekannter Weise
a) eine Verbindung der Formel II 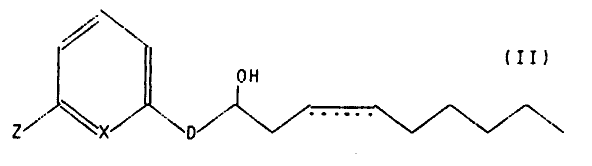 worin D die oben angegebene Bedeutung hat und .... eine Einfach-oder eine Doppelbindung und X Stickstoff darstellen und worin Z ein Bromatom oder ein Iodatom symbolisiert, nach Umsetzung mit n-Butyllithium mit einer Verbindung der Formel III
R¹-B-R⁴ (III),
worin R¹ und B die oben angegebenen Bedeutungen haben und R⁴ die Reste -CHO oder -COOCH₃ bedeutet, umsezt, oder
b) eine Verbindung der Formel IV, 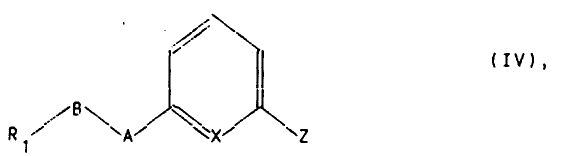 worin R¹, B, A und Z die oben angegebenen Bedeutungen haben und X Stickstoff bedeutet, mit einem Tri-n-butylstannan der Formel V, 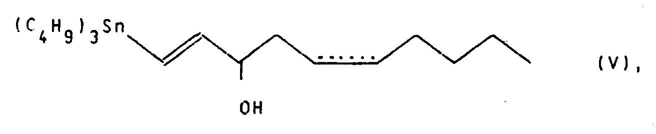 worin ..... eine Einfach- oder eine Doppelbindung darstellt, in Gegenwart eines Palladiumkatalysators umsetzt, oder
c) ein Phenylderivat der Formel VI, 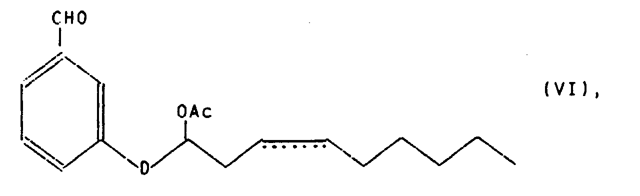 worin D die Gruppen 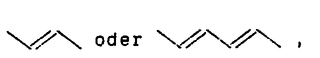 Ac eine Acylgruppe mit bis zu 8 C-Atomen und ... eine Einfach- oder eine Doppelbindung bedeuten, mit einer Grignardverbindung der allgemeinen Formel VII 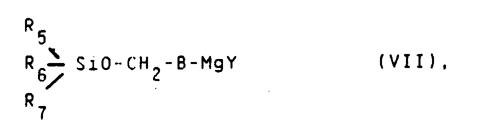 worin B die obengenannte Bedeutung besitzt, Y ein Chloratom oder ein Bromatom darstellt und die Substituenten R₅, R₆ und R₇ gleich oder verschieden sind und C₁-C₄-Alkylgruppen oder Phenylgruppen bedeuten umsetzt, die freie Hydroxygruppe acyliert den Silyletehr und gewünschtenfalls die Hydroxymethylverbindung zur Carboxylverbindung oxidiert, sowie vorhandene Acylgruppen abspaltet und die Carboxylgruppe verestert oder in ihre Salze überführt.
